# EUROPEAN PATENT APPLICATION

(11) **EP 2 128 621 A1**
(43) Date of publication of application: **02.12.2009**
(21) Application number: 07707909.3
(22) Date of filing: 01.02.2007
(51) Int. Cl.: G01N 33/574

(54) **METHOD OF DETECTING MAMMARY CANCER CELLS**

(71) Applicant: Oriental Yeast Co., Ltd., Tokyo 1748505 (JP)
(72) Inventor: KINUGASA, Masahiro, Nagahama-shi Shiga 526-0804 (JP); SUGIMOTO, Michiyo, Nagahama-shi Shiga 526-0804 (JP); UCHIDA, Koji, Nagahama-shi Shiga 526-0804 (JP)
(74) Representative: Daniels, Jeffrey Nicholas
(86) International application number: PCT/JP2007/051723
(87) International publication number: WO 2008/093427

(57) **Abstract**

The present invention provides a novel method for detecting mammary cancer cells which uses, as an index, an expression level of a specific gene in human mammary cancer tissue (or cells). The method is **characterized by** including (1) measuring an expression level of a gene having a specific nucleotide sequence in human mammary cancer tissue (or cells), (2) measuring an expression level of the gene in human normal mammary gland tissue (or cells), and (3) detecting mammary cancer cells on the basis of the difference between measurement values obtained in (1) and (2).

## Description

### Technical Field

The present invention relates to a novel method for detecting mammary cancer cells, more specifically, to a method for detecting mammary cancer cells which is based on comparing normal cells with mammary cancer cells in terms of the expression level of a marker gene having a specific nucleotide sequence.

### Background Art

In Japan, the number of patients suffering from mammary cancer has been being drastically increasing. This is the most popular cancer particularly for women. Mammary cancer is associated with a female hormone (estrogen) and thus, is often observed in women, for example, those who experience menarche at an early age, who experience menopause at a late age, whose age at first birth is high, or who are old and nulliparous. Such women have been exposed to estrogen for a long period of time, and are likely to develop mammary cancer. In addition, mammary cancer is also associated with, for example, obesity and fat-rich meals due to the increasing Western-style diet, since postmenopausal women, among others, produce estrogen in adipose tissue. Furthermore, changes in Japanese women's lifestyles (e.g., increased participation in society) are associated with increase in the number of patients suffering from mammary cancer.

Mammary cancer is roughly classified into three types; i.e., non-invasive cancer, invasive cancer and Paget disease. Lump-forming mammary cancer is in most cases invasive cancer, which is classified into general cancer (e.g., scirrhous cancer, papillotubular cancer and solid tubular cancer) and specific cancer (e.g., mucinous cancer).

There are no blood tests for specifically diagnosing early-stage mammary cancer and thus, palpation and X-ray image diagnosis are performed. However, even if these are combined with each other, the undetected rate is as high as 20%. Also, X-ray image diagnosis requires the skills of a medical specialist, which is inconvenient. Meanwhile, in preoperative or perioperative cytological diagnosis of mammary cancer, differential diagnosis must be made by pathologists. But, one problem involving such differential diagnosis is that the number of pathologists is not sufficient. Another problem is that its criteria are varied. Thus, there are no objective, simple detection/diagnosis methods which differentiate the data obtained in medical examination. PET diagnosis, capable of detecting cancer tissue with a diameter of 1 mm or smaller, has been performed in recent years, but requires large-scale equipment. Thus, it is very difficult to perform for simple medical examination.

The recent studies have revealed that cancer is a disease caused by genetic abnormality. In view of this, some patent literatures proposed detection methods for cancer cells which are based on the difference in expression level of a specific gene, for example, between cancer tissue and normal tissue (patent literatures 1 and 2).
Patent Literature 1: Japanese Patent Application Laid-Open (JP-A) No. 2003-284594
Patent Literature 2: JP-A No. 2003-284596

### Disclosure of Invention

### Problems to be Solved by the Invention

Conventionally known examination/ diagnosis methods, however, may require a long time for completion of examination or may give inaccurate data, and thus are not satisfactory methods. Under such circumstances, in order to be applied to diagnosis of early-stage cancer, rapid diagnosis, and evaluation/prediction of a select therapeutic method, keen demand has arisen for a simple, rapid detection method for cancer cells which uses genetic abnormality as an index.

### Means for Solving the Problems

In view of the above-described problems, the present inventors conducted extensive studies and have found that a specific marker gene is different in expression level between mammary and normal tissues, and can be applied to detection of mammary cancer cells. The present invention has been accomplished on the basis of the finding. Accordingly, means for solving the problems are as follows.
<1> A method for detecting mammary cancer cells, including:
   detecting mammary cancer cells on the basis of a difference in expression level of a marker gene between human mammary cancer tissue (or cells) and normal mammary gland tissue (or cells).
<2> The method according to < 1 > above, wherein the expression level of the marker gene is indicated by an amount of mRNA transcribed therefrom.
<3> The method according to < 1 > or < 2 > above, wherein the marker gene is at least one selected from the group consisting of genes having nucleotide sequences of GenBank accession Nos. BC071926, AC005028, AY424280, BC070036, BC041424, BC050454, AC068213, BC016981, AL138808, AJ505757, BC033015, BC015523, AL110326 and BC065198 and homologs thereof.
<4> The method according to any of < 1 > to < 3 > above, wherein the expression level of the marker gene in the mammary cancer tissue (or cells) is twice or more or 1/2 or less than that in the normal mammary gland tissue (or cells).

### Effects of the Invention

A method of the present invention for detecting mammary cancer cells enables mammary cancer cells to be rapidly and simply detected on a genetic level, which leads to early detection and rapid cure of mammary cancer.

### Brief Description of Drawings

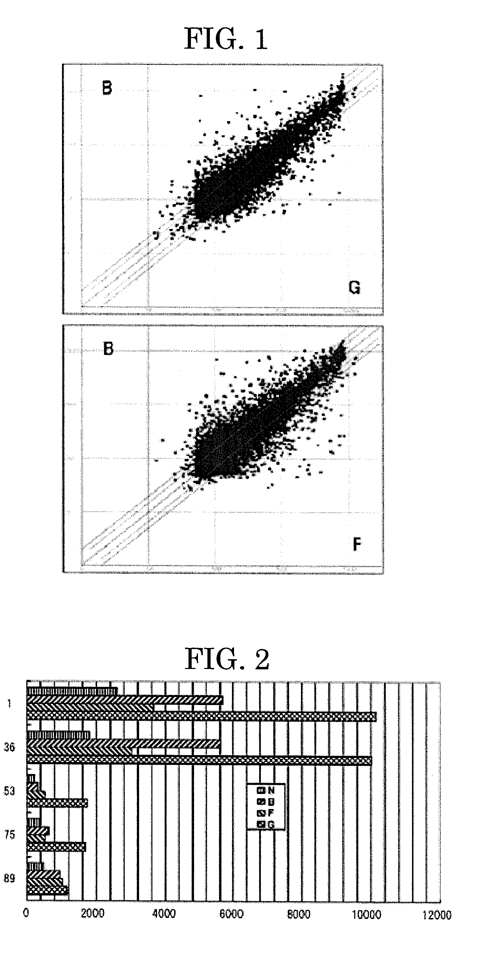

FIG.1 is a graph obtained by plotting peaks of expression level ranges in each of one normal mammary gland tissue (sample #N) and three human mammary cancer tissues (samples #B, #F and #G), wherein the vertical axis corresponds to expression levels in sample #B (signal intensities of waveform data) and the horizontal axis expression levels in sample #F (or #G) (signal intensities of waveform data).
FIG. 2 is a graph of expression level ranges of genes indentified, wherein the vertical axis corresponds to ID Nos. of the genes and the horizontal axis expression levels (signal intensities of waveform data).

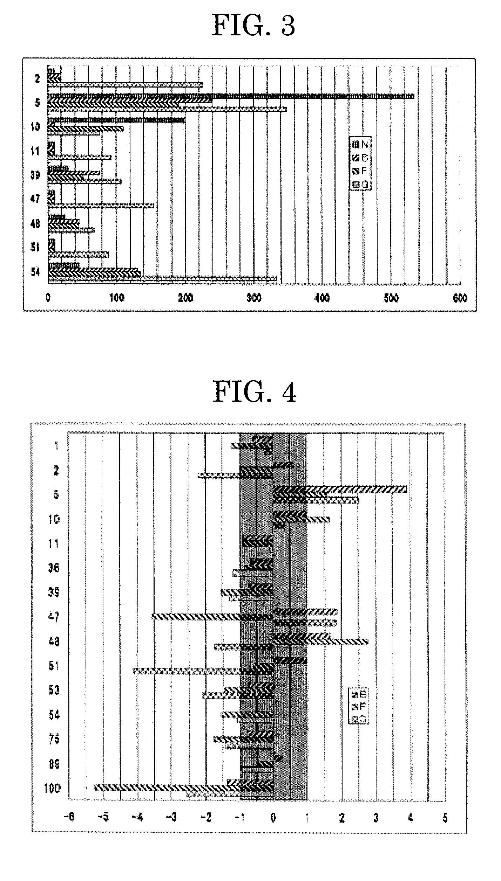

FIG. 3 is a graph of expression level ranges of genes indentified, wherein the vertical axis corresponds to ID Nos. of the genes and the horizontal axis expression levels (signal intensities of waveform data).
FIG. 4 is a graph of comparison of gene expression level ranges of one normal mammary gland tissue (sample #N) and three human mammary cancer tissues (samples #B, #F and #G), wherein the vertical axis and the horizontal axis correspond to ID Nos. of the genes and relative gene expression levels (ΔΔCt values), respectively, wherein the shaded region indicates a range narrower than that twice the theoretical expression level range, and wherein the gene of ID No. 100 of the vertical axis is gene c-erbB2 used as a control.

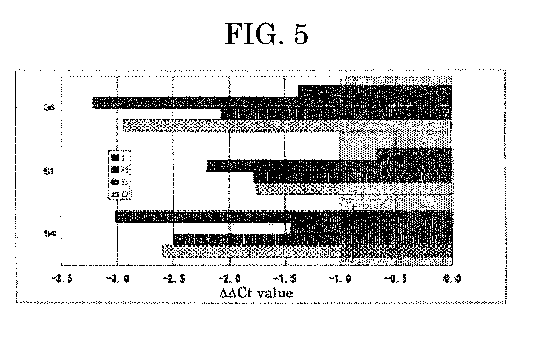

FIG. 5 is a graph of comparison of gene expression level ranges of one normal mammary gland tissue (sample #N) and four human mammary cancer tissues (samples #D, #E, #H and #I), wherein the vertical axis and the horizontal axis correspond to ID Nos. of the genes and relative gene expression levels (ΔΔCt values), respectively, and wherein the shaded region indicates a range narrower than that twice the theoretical expression level range.

### Best Mode for Carrying Out the Invention

A method of the present invention for detecting mammary cancer cells is characterized by using as an index the difference in expression level of a specific marker gene between mammary cancer cells and normal cells. In the present invention, the marker gene is a gene with which whether or not a certain cell is a mammary cancer cell can be judged by comparing the cell with a normal cell in terms of its expression level.

The method of the present invention for detecting mammary cells is **characterized in that** the expression level of the marker gene is indicated by the amount of mRNA thereof. More specifically, the method of the present invention for detecting mammary cancer cells is characterized by including extracting total RNA from mammary cancer tissue-derived cells and normal mammary gland tissue-derived cells, and comparing the mammary cancer tissue-derived cells with the normal mammary gland tissue-derived cells in terms of the amount of mRNA transcribed from the marker gene. Gene expression analysis employable for measuring the amount of mRNA transcribed from the gene is, for example, high coverage expression profiling (HiCEP) analysis, DNA microarray, quantitative reverse transcription PCR (quantitative RT-PCR) and northern hybridization. In addition, the present invention can employ in situ hybridization; i.e., gene expression analysis which measures the amount of mRNA without extracting total RNA from cells. Also, in order to enhance detection accuracy, two or more of the above-listed analyses can be used in combination. Meanwhile, one exemplary method for measuring the amount of a translated product of the gene is a method for measuring the amount of protein encoded by the gene. Specifically, the amount of a specific protein can be measured by, for example, immunoassays using an antibody specific for the protein (e.g., ELISA, western blotting and RIA), two-dimensional electrophoresis and high-performance liquid chromatography. The antibody specific for the protein encoded by the gene can be prepared with a routine method using it as an immunizing antigen.

No particular limitation is imposed on the marker gene used in the present invention, so long as it exhibits a significant difference in expression level between mammary cancer cells and normal cells. Examples thereof include 14 genes of GenBank accession Nos. BC071926, AC005028, AY424280, BC070036, BC041424, BC050454, AC068213, BC016981, AL138808, AJ505757, BC033015, BC015523, AL110326 and BC065198 and homologs thereof, and at least one selected from the group consisting thereof can be employed. Preferably, there is used at least one selected from the group consisting of 11 genes of GenBank accession Nos. BC071926, AC005028, AY424280, BC070036, BC050454, AC068213, AJ505757, BC033015, BC015523, AL110326 and BC065198 and homologs thereof. Notably, in the present invention, the term "homolog" refers collectively to a group of genes "whose GenBank accession Nos. are different but which are identical." This is because, in GenBank, different researchers may register the same gene at different timings under different fields and names, or single-nucleotide-mutated genes may be registered as novel genes and thus, a single gene has different several accession numbers.

The method of the present invention for detecting mammary cancer cells is characterized by using a marker gene which shows a significant difference in expression level between mammary cancer cells and normal cells. Here, the term "expression level" refers to an amount of mRNA transcribed from the marker gene or an amount of protein translated from the mRNA. Regarding the difference in expression level therebetween, the expression level in the mammary cancer cells is preferably 1.5 or higher or 2/3 or lower, more preferably 2 or higher or 1/2 or lower, with that in the normal cells being regarded as 1. When the difference in expression level of the marker gene between the normal cells and the mammary cancer cells deviates from the above ranges, it is difficult to determine whether or not target cells are cancer cells, which is not preferred.

Target mammary cancer may be any type of mammary cancer such as ductal mammary cancer (e.g., papillotubular cancer, solid tubular cancer and scirrhous cancer), lobular cancer, specific mammary cancer (e.g., mucinous cancer, medullary cancer and tubular cancer) and Paget disease. Preferred target mammary cancer is scirrhous cancer, lobular cancer or solid tubular cancer.

### Example 1

The present invention will next be described by way of Examples, which should not be construed as limiting the present invention thereto. In the Examples, human mammary cancer tissue and normal mammary gland tissue were analyzed for gene expression level (RNA transcription level) through high coverage expression profiling analysis (HiCEP technique) (Nucleic Acids Res., 2003, vol. 31(16), p. 94) - one known technique for gene expression analysis. And, the mammary cancer tissue was compared with the normal mammary gland tissue in terms of expression levels of the genes thereof.

Specifically, one normal mammary gland tissue (sample #N) and three human mammary cancer tissues (samples #B, #F and #G) (Table 1) were treated using an RNeasy kit (product of QUIAGEN Co.) with a routine method for the kit, to thereby extract total RNA samples therefrom. Then, using a Super Script First Strand Synthesis System for RT-PCR (product of Invitrogen Corporation), reverse transcription was carried out using each total RNA sample (1 µg) as a template. The reaction product resulting from reverse transcription was reacted at 16°C for 2 hours with DNA polymerase I (80 units) (product of Invitrogen Corporation), RnaseH (4 units) (product of Invitrogen Corporation), and E. Coli DNA ligase (40 units) (product of Invitrogen Corporation). The thus-obtained double-stranded cDNA was reacted at 37°C for 4 hours with restriction enzyme MseI (40 units) (product of NEW ENGLAND BioLabs Inc.) and restriction enzyme MspI (50 units) (product of TAKARA BIO INC.). The resultant DNA fragments were ligated with adapter sequences at both ends thereof. Subsequently, selective-PCR was performed using as a template the thus-prepared adapter sequence-ligated DNA fragments. Thereafter, the reaction product was analyzed through capillary electrophoresis. The obtained waveform data were used to determine gene expression levels, to compare the samples with one another in terms of gene expression level, and to classify a pattern of expressed genes, whereby clustering data (peaks of gene expression level ranges) were obtained.

**Table 1**

| Sample No. | Sex | Type of mammary cancer | Histological atypism | HER2 expression* | ER expression* |
|---|---|---|---|---|---|
| B | Female | Scirrhous cancer | NG1 | + | + |
| F | Female | Scirrhous cancer | NG2 | +++ | - |
| G | Female | Lobular cancer | NG3 | + | + |
| N | Female | Normal mammary gland | - | - | - |

| | | | | | |
|---|---|---|---|---|---|
| *: gene expression level in cancer tissue | | | | | |

The three human mammary cancer tissue samples were analyzed for expression levels of all the genes, and were plotted on graphs (FIG. 1). Notably, on the graphs of FIG. 1, data located farther from the diagonal line of each graph indicate that the genes corresponding to the data exhibit greater differences in expression level between the normal mammary gland tissue sample and the human mammary cancer tissue samples. Also, it is obvious that, similar to the data plotted corresponding to the gene expression levels of mammary cancer tissue samples B and G (or F) shown in FIG. 1, there are also plotted data corresponding to the genes whose expression levels are different between normal mammary gland tissue sample N and mammary cancer tissue samples B and G (or F). On the basis of these graphs, 34 genes of the mammary cancer tissue samples were selected whose expression levels were considerably different from those of the genes of the normal mammary gland tissue sample (i.e., which were located farther from the diagonal line), followed by sequencing. As a result, 20 genes were found to be of unknown function, 10 genes of known function, and 4 genes novel. Among them, regarding 10 genes of known function, 2 genes of unknown function, and 2 novel genes (14 genes in total), the peaks indicating their expression levels in the samples are shown in FIGs. 2 and 3.

Also, the data obtained through sequencing of the 14 genes clearly indicates that these genes are those registered in GenBank as accession Nos. BC071926, AC005028, AY424280, BC070036, BC041424, BC050454, AC068213, BC016981, AL138808, AJ505757, BC033015, BC015523, AL110326 and BC065198 and homologs thereof. The names of the genes specified by these accession Nos. are listed in the below Table 2.

**Table 2**

| ID No. | Gene name | GenBank accession No. |
|---|---|---|
| 1 | Ribosomal protein S24 | BC071926 |
| 2 | GLI-Kruppel family member GLI3 | AC005028 |
| 5 | Adiponectin receptor 2 | AY424280 |
| 10 | Epsin 2 | BC070036 |
| 11 | EST, clone IMAGE: 3907313 | BC041424 |
| 36 | Dishevelled, dsh homolog 1 | BC050454 |
| 39 | Novel gene fragment, chr. 15 | AC068213 |
| 47 | Eph receptor A4 | BC016981 |
| 48 | Novel gene fragment, chr. 20 | AL138808 |
| 51 | G protein-coupled receptor 26 | AJ505757 |
| 53 | RAS p21 protein activator 1 | BC033015 |
| 54 | Glutathione S-transferase A4 | BC015523 |
| 75 | EST, moderately similar to zinc finger protein 195 | AL110326 |
| 89 | LUC-7 like (S. cerevisiae) | BC065198 |

### Example 2

In this Example, quantitative RT-PCR was performed on the 14 genes, whose expression levels had been found to be different from those of the genes of the normal mammary gland tissue sample in Example 1, and their expression levels in the mammary cancer tissue samples were compared with those in the normal mammary gland tissue sample. Specifically, total RNA samples were extracted from the tissue samples with a routine method. Then, reverse transcription was performed using as a template each total RNA sample (1 µg), to thereby synthesize single-stranded cDNA fragments complementary to mRNA fragments contained in the total RNA sample. Subsequently, using the thus-synthesized single-stranded cDNA fragment serving as a template and SYBER Green PCR Master (product of TOYOBO CO., LTD.), real-time PCR was performed through a routine method with a real-time PCR device (Light Cycler, product of Roche Diagnostics K.K.). On the basis of the obtained gene amplification curve, the genes of interest in the tissue samples were analyzed for threshold cycle values (Ct values), and the Ct values were primarily corrected with the Ct value of the internal standard gene. Thereafter, the primarily corrected Ct values (ΔCt values) of the mammary cancer tissue samples were secondarily corrected with the primarily corrected Ct values (ΔCt values) of the normal (N) mammary gland tissue sample. The secondarily corrected Ct values (ΔΔCt values) were relative gene expression levels obtained by correcting the expression levels of the genes of interest in the mammary cancer tissue samples with those in the normal mammary gland tissue sample.

Subsequently, using the ΔΔCt values of the 14 genes which had been selected in Example 1, mammary cancer tissue samples #B, #F and #G were compared with one another in terms of gene expression level range. The results are shown in FIG. 4. As is clear from FIG. 4, the gene of ID No.11 was found to show an expression level range narrower than 2. Also, in the genes of ID Nos. 47 and 48, increase or decrease in expression level was found to depend greatly on the type of the sample used. In contrast, among the 14 genes selected in Example 1,11 genes registered in GenBank as accession Nos. BC071926, AC005028, AY424280, BC070036, BC050454, AC068213, AJ505757, BC033015, BC015523, AL110326 and BC065198 and homologs thereof were found to exhibit significant differences in expression level between the mammary cancer tissue samples and the normal mammary gland tissue sample and to exhibit reproducibility thereof, clearly indicating that they were useful as a marker gene for detecting mammary cancer cells. Notably, the three genes of ID Nos. 11, 47 and 48 (registered in GenBank as accession Nos. BC041424, BC016981 and AL138808) and homologs thereof are also genes whose expression levels are different between the mammary cancer tissue samples and the normal mammary gland tissue sample. These three genes, therefore, can be suitably used for a method of the present invention for detecting mammary cancer cells.

### Example 3

In this Example, quantitative RT-PCR was performed on three genes of the 11 genes, whose expression levels had been found to be particularly different from those of the genes of the normal mammary gland tissue sample in Example 2, and their expression levels in mammary cancer tissue samples were compared with those in the normal mammary gland tissue sample of Example 2. The mammary cancer tissue samples used were mammary cancer tissue samples #D, #E, #H and #I (Table 3) different from those used in Example 2. Specifically, total RNA samples were extracted from the tissue samples with a routine method. Then, reverse transcription was performed using as a template each total RNA sample (1 µg), to thereby synthesize single-stranded cDNA fragments complementary to mRNA fragments contained in the total RNA sample. Subsequently, using the thus-synthesized single-stranded cDNA fragment serving as a template and SYBER Green PCR Master (product of TOYOBO CO., LTD.), real-time PCR was performed through a routine method with a real-time PCR device (Light Cycler, product of Roche Diagnostics K.K.). On the basis of the obtained gene amplification curve, the genes of interest in the tissue samples were analyzed for threshold cycle values (Ct values), and the Ct values were primarily corrected with the Ct value of the internal standard gene. Thereafter, the primarily corrected Ct values (ΔCt values) of the mammary cancer tissue samples were secondarily corrected with the primarily corrected Ct values (ΔCt values) of the normal (N) mammary gland tissue sample. The secondarily corrected Ct values (ΔΔCt values) were relative gene expression levels obtained by correcting the expression levels of the genes of interest in the mammary cancer tissue samples with those in the normal mammary gland tissue sample.

**Table 3**

| Sample No. | Sex | Type of mammary cancer | Histological atypism | HER2 expression* | ER expression* |
|---|---|---|---|---|---|
| D | Female | Scirrhous cancer | NG1 | ++ | + |
| E | Female | Solid tubular cancer | NG2 | - | +++ |
| H | Female | Lobular cancer | NG3 | + | +++ |
| I | Female | Scirrhous cancer | NG1 | ++ | + |
| N | Female | Normal mammary gland | - | - | - |

| | | | | | |
|---|---|---|---|---|---|
| *: gene expression level in cancer tissue | | | | | |

Subsequently, using the ΔΔCt values of the three genes (ID Nos. 36, 51 and 54) of the 11 genes which had been selected in Example 2, mammary cancer tissue samples #D, #E, #G and #1 were compared with one another in terms of their expression levels. The results are shown in FIG. 5. As is clear from FIG. 5, among the 11 genes selected in Example 2, the three genes registered in GenBank as accession Nos. BC050454, AJ505757 and BC015523 and homologs thereof were found to exhibit significant differences in expression level between the mammary cancer tissue samples and the normal mammary gland tissue sample and to exhibit reproducibility thereof, clearly indicating that they were useful as a marker gene for detecting mammary cancer cells. In addition, the eight genes of BC071926, AC005028, AY424280, BC070036, AC068213, BC033015, AL110326 and BC065198 and homologs thereof, selected in a manner similar to that employed in selecting the three genes of BC050454, AJ505757 and BC015523 and homologs thereof, are sufficiently expected to be a useful marker gene for detecting mammary cancer cells.

### Industrial Applicability

Use of a gene(s) of the present invention realizes a high-sensitive, objective, simple and rapid detection method for mammary cancer cells, which has not conventionally been attained. Also, in the future, the present invention is thought to be applied to, for example, gene markers for judging mammary cancer to be excised, markers for detecting early-stage mammary cancer, markers for judging malignancy of mammary cancer, and markers for predicting prognosis of mammary cancer.

## Claims

1. A method for detecting mammary cancer cells, comprising:
detecting mammary cancer cells on the basis of a difference in expression level of a marker gene between human mammary cancer tissue (or cells) and normal mammary gland tissue (or cells).

2. The method according to claim 1, wherein the expression level of the marker gene is indicated by an amount of mRNA transcribed therefrom.

3. The method according to claim 1 or 2, wherein the marker gene is at least one selected from the group consisting of genes having nucleotide sequences of GenBank accession Nos. BC071926, AC005028, AY424280, BC070036, BC041424, BC050454, AC068213, BC016981, AL138808, AJ505757, BC033015, BC015523, AL110326 and BC065198 and homologs thereof.

4. The method according to any of claims 1 to 3, wherein the expression level of the marker gene in the mammary cancer tissue (or cells) is twice or more or 1/2 or less than that in the normal mammary gland tissue (or cells).
